# EUROPÄISCHE PATENTSCHRIFT

(11) **EP 2 967 934 B1**
(45) Veröffentlichungstag und Bekanntmachung des Hinweises auf die Patenterteilung: **01.03.2017**
(21) Anmeldenummer: 14711443.3
(22) Anmeldetag: 13.03.2014
(51) Int. Cl.: A61F 2/90, A61F 2/856, A61L 31/14

(54) **BIORESORBIERBARER STENT**
BIORESORBABLE STENT
ENDOPROTHÈSE BIORÉSORBABLE

(30) Priorität: 16.03.2013 DE 102013004625
(43) Veröffentlichungstag der Anmeldung: 20.01.2016
(73) Patentinhaber: Albert-Ludwigs-Universität Freiburg, 79104 Freiburg (DE)
(72) Erfinder: HEHRLEIN, Christoph, 79104 Freiburg i. Br (DE)
(74) Vertreter: Rösler, Uwe
(86) Internationale Anmeldenummer: PCT/EP2014/000672
(87) Internationale Veröffentlichungsnummer: WO 2014/146769

(56) Entgegenhaltungen:
- WO-A1-2008/137220
- WO-A2-2009/009376
- DE-A1-102010 027 124
- US-A1- 2012 150 275

## Beschreibung

### Technisches Gebiet

Die Erfindung bezieht sich auf einen Stent mit einem röhrchenförmigen, aus miteinander verbundenen Stentstreben bestehenden Gittergerüst, das gesamtheitlich aus einem bioresorbierbaren Material gefertigt ist und aus einer komprimierten ersten Raumform in eine radial aufgeweitete, formstabile rohrförmige zweite Raumform überführbar ist.

### Stand der Technik

Stents bezeichnen in der Gefäßmedizin medizinische Implantate zur mechanischen Aufweitung und Stützung von Hohlorganen, insbesondere von arteriellen Blutgefäßen. Zu Zwecken einer möglichst schonenden Implantation, vorzugsweise mittels Katheterunterstützer, minimalinvasiver Operationstechniken, bestehen Stents typischerweise aus einem plastisch verformbaren, zumeist aus Metall oder einer Metalllegierung gefertigten Gittergerüst in Röhrchenform, dessen Röhrchendurchmesser von einem kleinen, ersten Durchmesser, bei dem eine Patientenschonende Einführung und intrakorporale Positionierung des Stents möglich ist, auf einen zweiten, größeren Röhrchendurchmesser aufweitbar ist, bei dem der Stent seine therapeutische Wirkung zur Gefäßstützung und radialen Gefäßaufweitung, insbesondere an stenosierten Gefäßwandabschnitten, entfaltet.

Neben der erwünschten therapeutischen Stentwirkung können mit der intrakorporalen und zumeist dauerhaften Implantation von Stents auch unerwünschte Nebenwirkungen auftreten, die von körpereigenen Abwehrreaktionen auf den Stent als Fremdkörper herrühren. Durch den kraftbeaufschlagten mechanischen Kontakt zwischen der radial aufgeweiteten, röhrchenförmigen Gitterstruktur des Stents und der Gefäßwand sind Gewebereizungen unvermeidbar, die je nach Ausprägung Entzündungsreaktionen hervorrufen, die zur Bildung von Thrombosen oder Rezidivstenosen führen können. Zur Begegnung derartiger medizinischer Komplikationen sind Stents mit Beschichtungen und/oder mit Einlassungen in Form von Poren bekannt, die aus biokompatiblen mit Medikamenten versetzten Polymeren oder Keramiken bestehen, wodurch Medikamente über Diffusion in die Gefäßwand abgegeben werden können. In der Druckschrift WO 2012/057976 ist ein Stent mit Einlassungen in den Stentstreben beschrieben, die ein medikamentenhaltiges Polymer enthalten.

Durch eine dauerhafte Implantation von Stents besteht somit ein erhöhtes Thrombenrisiko, zu dessen Einschränkung eine dauerhaft begleitende antithrombozytäre Verabreichung von Medikamenten zumindest empfehlenswert ist.

Gleichwohl das Gittergerüst eines Stents, aufgrund seiner offenmaschig ausgebildeten Gitterstruktur stoff- und fluidurchlässig ist, stellen Stents, die in Gefäßbereichen zu implantierend sind, an denen eine Gefäßabzweigung vorhanden ist, die von einem Teil der offenmaschigen Gitterstruktur überdeckt wird, signifikante Strömungshindernisse dar. Um dieses Problem zu beseitigen sind spezielle Stents für den Einsatz an Haupt- und Seitenästen von menschlichen Gefäßen entwickelt worden. Aus der Druckschrift US 2012/0209368 A1 ist ein Stentsystem sowie ein Verfahren zu dessen Implantation zu entnehmen, bei dem der von einem Gefäßhauptast abgehende Gefäßseitenast durch einen separaten Stent zu einem längs des Hauptastes im Bereich des abgehenden Seitenastes befindlichen Stent, aufgeweitet wird. Der Nachteil dieser speziellen Seitenast-Stentsysteme ist jedoch, dass das großvolumige Implantat zu einer erheblichen Fremdkörperreaktion und damit häufig zu Rezidivstenosen im behandelten Gefäßbereich führt.

Alternative Stentdesigns mit großen Löchern oder Aussparungen, so genannten "open cell designs" oder "Fenestrierungen", die bei einem Stent realisiert sind, der in der Druckschrift EP 2 497 444 A1 beschrieben ist, um einen besseren Zugang von einem Hauptgefäßast zu entsprechenden Seitenästen zu schaffen, haben den Nachteil, dass es dauerhaft zu Gefäßeinengungen durch Gewebeprotrusionen genau an den Stellen kommt, an welchen der Stentkörper ein großes Loch oder die Fenestrierung aufweist.

Die mit einer dauerhaften Stentimplantation verbundenen Nachteile können mit einer seit einiger Zeit bekannten und im Einsatz befindlichen neuen Gattung von Stents zumindest reduziert werden, die aus bioresorbierbaren, bioabsorbierbaren oder biokorrodierbaren Materialien bestehende Stentstreben aufweisen, die sich vollständig innerhalb des Körpers aufzulösen vermögen und dadurch den Zeitraum der durch den Stent bedingten körperlichen Belastungen zumindest begrenzen.

In der Druckschrift WO 2009/155206 A2 ist ein derartiger bioresorbierbarer Stent aus einem bioresorbierbaren Polyester offenbart, der sukzessiv im Körper degradiert bzw. sich mit der Zeit weitgehend vollständig auflöst. In der Druckschrift WO 2008/118607 A2 wird ein aus einer Magnesium-Legierung bestehender Stent erläutert, der ebenfalls Eigenschaften einer Degradation mittels Bioresorption oder Biokorrosion aufweist.

Die Verwendung von sich auflösenden, bioresorbierbaren Stents führt zum einen zur Verbesserung von auftretenden Biokompatibilitätsproblemen von bisher bekannten nicht degradierbaren Stentmaterialien sowie zu einer zeitlichen Begrenzung von körperlichen, Thrombosen erzeugenden Abwehrreaktionen. Zudem ist eine Entnahme von implantierten Stents im Wege eines operativen Eingriffes, selbst im Rahmen eines nur minimalinvasiven Eingriffes, nicht mehr nötig.

Dennoch vermag auch der Einsatz derartiger Stents die Problematik bei der Implantation innerhalb eines Hohlorgans im Bereich wenigstens einer Gefäßabzweigung nicht zu lösen, zumal auch in diesem Fall ein in einem arteriellen Hauptgefäß im Bereich von Gefäßabzweigungen oder Gefäßabgängen implantierter Stent die Gefäßabgänge durch die Stentstreben verlegt und damit nachhaltigen Einfluss auf die Strömungsdynamik in diesem Gefäßbereich nimmt.

Der WO 2009/009376 A2 ist ein Stent mit einer bioresorbierbaren Membran zu entnehmen. Der Stent besteht aus einer röhrchenförmigen Gitterstruktur, die aus einer Vielzahl meanderförmig ringförmig ausgebildeten Stentstreben zusammengesetzt ist, die jeweils über Verbindungsstreben miteinander verbunden sind. Das sich daraus ergebende Gittergerüst des Stents schließt einen Flächenbereich auf der dem Stent zuordenbaren Mantelfläche ein, innerhalb dem die Gitterstruktur von der übrigen Gitterstruktur des Stents abweicht. So sind die innerhalb des Flächenbereiches befindlichen Stentstreben derart angeordnet und ausgebildet, so dass sie unter Ausbildung einer seitlich zum Stent orientierten und sich kragen- oder ebenfalls röhrchenförmig ausbildenden Seitenstentstruktur aufgeweitet werden können. Gleichwohl die Stentstreben innerhalb wie außerhalb des Flächenbereiches von einer bioresorbierbaren zusätzlichen Membran umgeben sind, stellt der letztlich implantierte Stent einen dauerhaft intrakorporalen, den betreffenden Gefäßbereich irritierenden Fremdkörper dar.

Eine ähnliche Stentanordnung ist der Druckschrift WO 2009/009311 A2 zu entnehmen, die zu Zwecken der Implantation in einen Gefäßbereich mit einer Gefäßabzweigung über eine röhrchenförmige Gitterstruktur verfügt, die gleichfalls, wie im vorigen Fall, über einen von einem Umfangsrand begrenzten Flächenbereich verfügt, innerhalb dem eine abgewandelte Gitterstruktur zur Ausbildung einer Seitenstentstruktur vorgesehen ist, die zur Abstützung einer Gefäßabzweigung dient. Basierend auf der Erkenntnis, dass eine derartige zu Zwecken der Implantation innerhalb eines Gefäßabzweigungsbereiches ausgebildete Stentstruktur speziell im Übergangsbereich zwischen dem röhrchenförmigen Stent zur Stützung des Hauptgefäßes und dem sich abzweigenden Nebenstent zur Stützung der Gefäßabzweigung, d.h. im Bereich des vorstehend erwähnten Umfangsrandes, eine erhöhte mechanische Belastung erfährt, sieht die bekannte Stentanordnung im Bereich des Umfangsrandes Stentstrebenanteile vor, die aus bioresorbierbarem Material bestehen. Durch derartige, sich selbständig innerhalb des Körpers auflösende Stentstrebenanteile können mechanische Stressmomente im Bereich des Überganges zwischen Haupt- und Nebenast der Stentanordnung vermieden werden. Gleichwohl bleiben die mit den in den jeweiligen Gefäßbereichen dauerhaft verorteten Stentabschnitten verbundenen Nachteile, wie vorstehend ausgeführt, bestehen.

In vorteilhafter Weise bieten die in der Druckschrift DE 10 2010 027 124 A1 offenbarten Stents, die aus einer biologisch abbaubaren Magnesiumlegierung bestehen, die Möglichkeit einer restlosen intrakorporalen Auflösung. Die sich intrakorporal vollständig auflösenden Stents besitzen aufgrund unterschiedlicher Absorptionseigenschaften unterschiedliche Auflösungseigenschaften durch Variation der Legierungszusammensetzung (Kornfeinung) der Stentstreben. So kann die Korrosionsgeschwindigkeit derart beeinflusst werden, dass sich ein Implantatbereich im Bereich einer Bifokation bzw. einer Gefäßabzweigung schneller auflöst, als ein anderer Implantatbereich, so dass der Durchfluss im Seitengefäß gewährleistet ist.

Die Druckschrift US 8,109,991 B2 beschreibt einen Stent, der nicht vollständig resorbierbar ist, sondern lediglich bioresorbierbare Verbindungselemente zwischen einzelnen ringartigen Stentelementen aufweist. Die Verbindungselemente sollen sich auflösen, um eine verbesserte Anpassung des Stents an Bewegungen des Gefäßes, und damit eine Verringerung von Komplikationen, zu ermöglichen.

Die Druckschrift US 2003/0 199 993 A1 beschreibt ein bioresorbierbares Implantat, das über die Zeit unterschiedliche Auflösungsraten aufweist. Das Implantat weist hierzu einen Schichtaufbau aus unterschiedlichen Materialien auf, wobei in die einzelnen Schichten zusätzlich Partikel eingebracht sein können. Durch lokale Veränderung der Verteilung der Partikel können im Implantat lokal unterschiedliche Absorptionsraten eingestellt werden.

Die Druckschrift US 2012/0150275 A1 offenbart einen bioresorbierbaren Stent, der durch Aufdrucken eines "tintenartigen" Materials auf einem Trägerkörper erzeugt wird. Der besondere Vorteil des Verfahrens besteht darin, dass ein nahtloser Stent hergestellt werden kann.

Die Druckschrift DE 10 2005 018 356 A1 bezieht sich auf resorbierbare Implantate aus einem resorbierbaren Grundkörper und einer bioabbaubaren Beschichtung. Der Grundkörper besteht aus einem Metall, einer Metalllegierung, einem Metallsalz, einem Polymer oder Mischungen dieser Verbindungen. Dagegen besteht die bioabbaubare Beschichtung bevorzugt aus biodegradierbaren Polymeren und enthält ferner mindestens eine pharmakologische aktive Substanz.

### Darstellung der Erfindung

Der Erfindung liegt die Aufgabe zugrunde, einen Stent zur Implantation in einen intrakorporalen Gefäßbereich mit wenigstens einer Gefäßabzweigung derart weiterzubilden, so dass zum einen gewährleistet wird, dass der wenigstens eine abzweigende Gefäßkanal durch den implantierten Stent nicht überdeckt wird, um so eine negative Beeinflussung des Strömungsverhaltens, insbesondere im Bereich der Gefäßabzweigung, zu vermeiden. Zum anderen soll dafür Sorge getragen werden, dass der implantierte Stent keine oder nur geringfügige durch seine Fremdkörpereigenschaften bedingten Gewebeirritationen hervorrufen soll, so dass nachhaltig ausgeschlossen werden kann, dass die Gefahr von entzündungsinitiierten Thrombenbildungen ausgeschlossen oder zumindest signifikant reduziert werden kann. Damit verbunden gilt es, die Voraussetzung dafür zu schaffen, dass eine bislang therapiebedingte antithrombozitär wirkende Medikation bei Patienten überflüssig wird. Ein weitere Aspekt gilt der Vorsorge dafür, dass ausgeschlossen werden soll, dass sich im Wege der Zersetzung des Stents nicht vollständig aufgelöste Stentabschnitte vom übrigen Stentgerüst ablösen und als gefährliche Fremdkörper in die Blutbahn gelangen können. Schließlich soll es möglich sein den Stent möglichst einfach und damit kostengünstig herzustellen.

Die Lösung der der Erfindung zugrunde liegenden Aufgabe ist im Anspruch 1 angegeben. Den Erfindungsgedanken in vorteilhafter Weise weiterbildende

Merkmale sind Gegenstand der Unteransprüche sowie der weiteren Beschreibung, insbesondere unter Bezugnahme auf die Ausführungsbeispiele, zu entnehmen.

Die der Erfindung zugrunde liegende Idee geht von einem Stent mit einem röhrchenförmigen, aus miteinander verbundenen Stentstreben bestehenden Gittergerüst aus, das gesamtheitlich aus einem bioresorbierbaren Material gefertigt ist und aus einer komprimierten ersten Raumform in eine radial aufgeweitete, formstabile röhrchenförmige zweite Raumform überführbar ist. Mit dem vollständig aus bioresorbierbarem Material bestehenden Gittergerüst ist die Voraussetzung dafür geschaffen, dass eine dauerhafte, mit der Implantation eines Stents verbundene Reizung bzw. Irritation, der mit dem Stent in Kontakt stehenden Gewebebereiche ausgeschlossen werden kann, zumal die therapeutisch wirksame Lebensdauer eines derartigen Stent begrenzt ist. Durch geeignete Materialwahl zur Ausbildung der Stents und/oder Vorsehen eine die Bioresorption verlangsamenden Beschichtung auf das entsprechend gewählte Stentmaterial kann weitgehend genau die therapeutische Lebensdauer des Stents, in Größenordnungen von Jahren und/oder Monaten festgelegt werden. Die lösungsgemäße Modifikation eines derartigen, an sich bekannten, vollständig aus bioresorbierbarem Material gefertigten Stents, besteht darin, innerhalb wenigstens eines bestimmten, einstückig zusammenhängenden, d.h. von einem in sich geschlossenen Umfangsrand umfassten Flächenbereiches auf einer dem röhrchenförmigen Gittergerüst des Stents zuordenbaren Mantelfläche befindliche Stentstreben derart zu konditionieren, so dass sich diese Stentstreben im Wege der Bioresorption in einer kürzeren Zeitspanne nach der Implantation innerhalb eines Hohlorgans eines Patienten aufzulösen vermögen, als die Stentstreben außerhalb des wenigstens einen bestimmten Flächenbereiches. Auf diese Weise bildet sich nach vollständigem Auflösen der sich innerhalb des Flächenbereiches befindlichen Stentstreben eine seitliche Öffnung innerhalb des röhrchenförmigen Gittergerüstes des Stents aus, die in Form und Größe an eine Gefäßabzweigung innerhalb des Hohlorgans angepasst ist, in dem der Stent in geeigneter Weise implantiert ist.

Bei der Ausbildung und Konditionierung der innerhalb des Umfangsrandes befindlichen Stentstreben gilt es in Hinblick auf ihre vollständige Auflösung im Wege der Bioresorption darauf zu achten, dass sich keine Stentstrebenbestandteile, die noch nicht vollständig bioresorbiert sind, von den umliegenden, innerhalb des Flächenbereiches befindlichen Stentstreben, ablösen und so als Fremdkörper in die Blutbahn gelangen. Um derartige Vereinzelungserscheingungen auszuschließen, gilt es in lösungsgemäßer Weise Maßnahmen dahingehend zu treffen, so dass sich Stentstreben innerhalb des Flächenbereiches mit größtem Abstand zum Umfangsrand in einer kürzeren Zeit aufzulösen vermögen als jene Stentstreben, die nahe dem Umfangsrand sind oder an diesem unmittelbar angrenzen.

Lösungsgemäß nimmt hierzu der Massenanteil an bioresorbierbarem Material pro Strebenlänge der Stentstreben innerhalb des Flächenbereiches mit zunehmendem Abstand der Stentstreben zum Umfangsrand kontinuierlich oder graduell, d.h. stufenweise, ab. So weisen beispielsweise die mittig innerhalb des Flächenbereiches befindlichen Stentstreben kleinere Stentdurchmesser auf, als die Stentstreben innerhalb des Flächenbereiches, die in der Nähe des Umfangsrandes angeordnet sind oder unmittelbar an diesen angrenzen.

Alternativ oder in Kombination mit der vorstehenden Massnahme bietet es sich an, das unterschiedliche zeitliche Auflösungsvermögen der Stentstreben innerhalb des Flächenbereiches durch eine geeignete Variation der Schichtdicke einer auf den Stentstreben innerhalb des Flächenbereiches abgeschiedenen, bioresorbierbaren Materialschicht vorzunehmen. Wird beispielsweise der lösungsgemäß ausgebildete, bioresorbierbare Stent an den Stentstreben des Gittergerüstes unter Ausschluss jener Stentstreben innerhalb des Flächenbereiches mit einer ersten Materialschicht beschichtet, die vorzugsweise an den beschichteten Gittergerüstbereichen eine einheitliche Schichtdicke aufweist, so ermöglicht eine Abscheidung einer zweiten Materialschicht an den Stentstreben innerhalb des Flächenbereiches durch geeignete Schichtdickenvariation das vorstehend erläuterte unterschiedliche zeitliche Auflösungsvermögen der innerhalb des Flächenbereiches befindlichen Stentstreben. Hierbei gilt es, die Schichtdicke der wenigstens zweiten Materialschicht mit zunehmendem Abstand zum Umfangsrand kontinuierlich oder graduell zu reduzieren. Somit ist gewährleistet, dass sich jene Stentstreben innerhalb des Flächenbereiches am schnellsten durch Bioresorption vollständig auflösen, die am weitesten von dem, den Flächenbereich einbeschreibenden Umfangsrand beabstandet sind.

Der neuartige Stent vermag somit nach Auflösen der betreffenden, die Gefäßabzweigung zunächst überdeckenden Stentstreben, das stenosierte Hohlorgan zum einen radial aufzuweiten und zum anderen für einen freien seitlichen Zugang zu einem vom Hohlorgan abzweigenden Nebengefäß zu sorgen.

Da sich der lösungsgemäße Stent in seinem Ausgangszustand, d.h. im Zustand vor und während des Vorgangs der Implantation von bekannten Stents nicht oder nicht nennenswert unterscheidet, ist der Stent in der gleichen Weise mit den Mitteln minimalinvasiver Operationstechniken handzuhaben. Auch der Vorgang der radialen Aufweitung nach entsprechender intrakorporaler Positionierung des Stents, bspw. mittels eines Ballonkatheters, unterscheidet sich nicht vom Dilatieren bekannter Stents. Der Operateur hat bei der intrakorporalen Positionierung des Stents lediglich zusätzlich darauf zu achten, dass der Stent axial und in seiner Umfangsrichtung exakt gegenüber einem seitlichen Öffnungsbereich einer Gefäßabzweigung zu liegen kommt. Hierzu sieht der Stent zur Markierung des Flächenbereiches geeignet am Stent angebrachte röntgendichte Marker vor, die dem Operateur eine exakte Stent-Positionierung ermöglichen.

Da das vollständige Gittergerüst des das Hohlorgan aufweitenden Stents aus bioresorbierbarem Material gefertigt ist, löst sich der Stent und die damit verbundene Stützfunktion selbständig auf, so dass die mit einem dauerhaften Verbleib eines Stents innerhalb eines Gefäßes verbundenen Nachteile vermieden werden. Durch geeignete Wahl von Material, Form und Größe für die Ausbildung der das Gittergerüst zusammensetzenden Stentstreben lässt sich die maximale Verweildauer des aus bioresorbierbarem Material gefertigten Stents festlegen.

Um zu gewährleisten, dass die innerhalb des wenigstens einen vorstehend erläuterten Flächenbereiches befindlichen Stentstreben nach Implantation einer schnelleren Auflösung im Wege der Bioresorption unterliegen als das übrige Gittergerüst, bieten sich folgende alternative oder in Kombination anzuwendende Maßnahmen an:
Eine erste Möglichkeit für ein rasches Auflösen der innerhalb des Flächenbereiches vorgesehenen Stentstreben besteht darin, diese Stentstreben mit einem geringeren Massenanteil an bioresorbierbarem Material pro vorgegebener diskreter Strebenlänge auszubilden, als die mittel- und/oder unmittelbaren am Flächenbereich angrenzenden Stentstreben des übrigen Gittergerüstes.

Da die Bioresorptionsrate, d.h. das zeitliche Auflösungsvermögen, durch das bioresorbierbare Material selbst vorgegeben ist, löst sich der implantierte Stent über seine gesamte räumliche Erstreckung einheitlich schnell auf. Durch den geringeren Massenanteil pro Strebenlänge betreffend die innerhalb des Flächenbereiches vorgesehenen Stentstreben, vermögen sich diese in kürzerer Zeit vollständig aufzulösen, als die Stentstreben im übrigen Gittergerüstbereich. Eine mögliche Maßnahme, den Massenanteil pro Strebenlänge betreffend die innerhalb des Flächenbereiches befindlichen Stentstreben zu reduzieren, ist die Reduzierung des Stentstrebendurchmessers gegenüber den Stentstreben außerhalb des Flächenbereiches. Gleichsam ist es denkbar, die Stentstreben innerhalb des wenigstens einen Flächenbereiches hohl auszubilden, gegenüber einer massiven Stentstrebenausbildung im übrigen Gittergerüstbereich des Stents. Letztere Maßnahme ermöglicht die Ausbildung von Stentstreben mit einheitlichem Stentstrebendurchmesser im gesamten Gittergerüst des Stents, wodurch eine einheitliche Strukturstabilität über den gesamten Stent realisierbar ist.

Eine weitere alternative Maßnahme zur vollständigen Auflösung der innerhalb des wenigstens einen Flächenbereiches befindlichen Stentstreben in einer kürzeren Zeitspanne, sieht die Beschichtung des aus einheitlichem bioresorbierbarem Material bestehenden Gittergerüstes mit einer zusätzlich, die Bioresorbierbarkeit des Gittergerüstes verzögernden Materialschicht vor, mit Ausnahme der Stentstreben innerhalb des wenigstens einen Flächenbereiches. Zudem stellt die Beschichtung des Gittergerüstes mit Ausnahme jener Stentstreben, innerhalb des wenigstens einen vorgegebenen Flächenbereiches, mit einer die Bioresorbierbarkeit herabsetzenden Wirkung aufweisenden Materialschicht, keine verfahrenstechnisch aufwendige und kostspielige Maßnahme dar.

Selbstverständlich ist es möglich, beide vorstehenden Maßnahmen zu kombinieren, so dass ein Stent, der innerhalb des wenigstens einen Flächenbereiches über Stentstreben verfügt, die einen im Vergleich zum übrigen Stenbereich geringeren Massenanteil pro Strebenlänge aufweisen, zusätzlich mit einer, die Bioresorbierbarkeit des Materials herabsetzenden Wirkung bestehenden Materialschicht beschichtet wird, wobei die Stentstreben innerhalb des wenigstens einen Flächenbereiches von der Materialbeschichtung ausgenommen sind. Ein derartig konditionierter Stent verfügt über eine verlängerte Lebensdauer gegenüber den innerhalb des Flächenbereiches und damit unbeschichteten und zusätzlich gedünnt ausgebildeten Stentstreben, die sich nach einer entsprechend kurzen Implantationszeit vollständig aufzulösen vermögen und somit zu einer seitlichen Öffnung innerhalb des Gittergerüstes des Stents führen, der in dieser Form einen freien und ungehinderten Zugang zu einer Gefäßabzweigung gewährleistet, während das die Gefäßabzweigung unmittelbar umgebende Gefäßgewebe innerhalb des Hohlorgans von dem Gittergerüst des Stents abgestützt bzw. radial aufgeweitet bleibt.

Eine dritte Alternative zur individuellen Konfektionierung des Bioresorptionsverhaltens der innerhalb des wenigstens einen Flächenbereiches befindlichen Stentstreben gegenüber den Stentstreben des übrigen Gittergerüstes des Stents besteht in einer Beschichtung der Stentstreben, ausgenommen der Stentstreben innerhalb des wenigstens einen vordefinierten Flächenbereiches, mit wenigstens einer ersten bioresorbierbaren Materialschicht. Die Stentstreben innerhalb des wenigstens einen Flächenbereiches sind mit wenigstens einer zweiten bioresorbierbaren Materialschicht beschichtet, die derart konditioniert ist, dass sie in einem kürzeren Zeitraum vollständig bioresorbierbar ist als die erste bioresorbierbare Materialschicht.

Auch die vorstehende dritte Alternative kann mit der erstgenannten Maßnahme kombiniert werden, d.h. die innerhalb des wenigstens einen Flächenbereiches befindlichen Stentstreben verfügen, verglichen zu den Stentstreben im übrigen Stentbereich, über einen geringeren Massenanteil an bioresorbierbarem Material pro Strebenlänge und sind darüber hinaus mit der vorstehend genannten, wenigstens zweiten bioresorbierbaren Materialschicht, beschichtet.

Die wenigstens zweite bioresorbierbare Materialschicht muss nicht notwendigerweise aus einem zur ersten bioresorbierbaren Materialschicht unterschiedlichen bioresorbierbarem Material bestehen, vielmehr ist es möglich, die erste und zweite Materialschicht aus identischem bioresorbierbarem Material zu fertigen, jedoch ist in diesem Fall die Schichtdicke der zweiten bioresorbierbaren Materialschicht innerhalb des wenigstens einen Flächenbereiches kleiner zu wählen, als die Materialschicht der ersten bioresorbierbaren Materialschicht im übrigen Stentbereich. Auf diese Weise ist gewährleistet, dass sich die dünner ausgebildete zweite Materialschicht nach entsprechender Implantation in einem kürzeren Zeitraum vollständig durch Bioresorption auflöst als die dicker ausgebildete erste bioresorbierbare Materialschicht.

Selbstverständlich sind Realisierungsformen des lösungsgemäß ausgebildeten Stents möglich, in denen sich die wenigstens erste und zweite Materialschicht durch die Materialart voneinander unterscheiden.

Eine bevorzugte Ausführungsform des Stents verfügt über ein röhrchenförmiges Gittergerüst, das über eine einheitliche Gitterstruktur verfügt, d.h. die Stentstreben weisen ein einheitliches Geflecht- oder Anordnungsmuster auf und umschließen mit einer geometrisch periodischen Wiederkehr jeweils offene Gitterstrukturmaschen, die vorzugsweise über eine einheitliche Maschenform verfügen. Aus ökonomischen Gründen sowie auch aus Gründen des Rückgriffes auf bekannte und bewährte Herstellungstechniken sind sämtliche, das Gittergerüst bildende Stentstreben aus einem einheitlichen bioresorbierbarem Material gefertigt und können je nach Fertigungsart einstückig miteinander verbunden sein oder in Form eines Geflechtes zur Ausbildung eines geflechtartigen Gittergerüstes gefügt bzw. verwoben sein.

Die Form und Größe des röhrchenförmigen Stents sind unter Maßgabe von Form und Größe des zu therapierenden Hohlorgans zu wählen. Dies gilt insbesondere auch für die Anzahl der längs der Mantelfläche des röhrchenförmigen Stents vorzusehenden Flächenbereiche sowie deren Form und Größe, die an die seitlich vom Hohlorgan abführenden Gefäßabzweigungen entsprechend zu wählen sind. So kann in Abhängigkeit von Form und Größe einer Gefäßabzweigung der den wenigstens einen Flächenbereich umschließende Umfangsrand kreisförmig, elliptisch, oval sein oder auch n-eckige Formen aufweisen, wobei n eine natürliche Zahl > = 3 ist. Der Umfangsrand kann als virtuelle, d.h. körperlich nicht in Erscheinung tretende Begrenzungslinie ausgebildet sein, dies ist der Fall, wenn das Gittergerüst über eine homogen durchgängige Gitterstruktur verfügt, wobei die Stentstreben beim Übergang in den wenigstens einen Flächenbereich einstückig, durchgängig ausgeführt sind. Ebenso kann der Flächenbereich von einem Umfangsrand in Form einer in sich geschlossenen Strebenstruktur umgeben sein, die allerdings derart ausgebildet ist, dass sie eine radiale Aufweitung des Stents ermöglicht.

Selbstverständlich ist es möglich, die vorstehenden Maßnahmen bezüglich der Materialschichtabscheidung sowie der unterschiedlichen Ausbildung des Massenanteils pro Strebenlänge der Stentstreben miteinander zu kombinieren.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a, b, c: schematisierte Darstellungen eines lösungsgemäß ausgebildeten Stents,
- Fig. 2a, b, c: schematisierte Darstellungen möglicher Realisierungsformen zur Ausbildung von Stentstreben innerhalb des wenigstens einen Flächenbereiches.

### Wege zur Ausführung der Erfindung, gewerbliche Verwendbarkeit

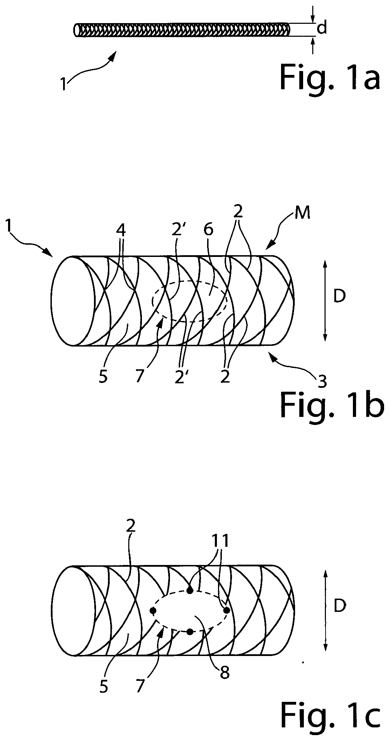

In Fig. 1a ist eine stark schematisierte Darstellung eines radial nicht aufgeweiteten röhrchenförmigen Stents 1 mit einem Stentdurchmesser d gezeigt. Figur 1b zeigt den gleichen Stent in radial aufgeweiteter Form mit einem Stentdurchmesser D > d. Der Stent 1 weist ein aus Stentstreben 2 gefertigtes Gittergerüst 3 auf, siehe Fig. 1b, das gesamtheitlich aus einem bioresorbierbaren Material besteht. Die einzelnen, das Gittergerüst 3 zusammensetzenden Stentstreben 2 sind an Verbindungsstellen 4 lose oder fest miteinander verbunden und schließen jeweils offen ausgebildete Gittermaschen 5 ein. Die einzelnen, das Gittergerüst 4 zusammensetzenden Stentstreben 2 bestehen vorzugsweise aus einem bioresorbierbaren Metall oder einer bioresorbierbaren Metalllegierung, wobei das Metall eines der nachstehenden Metalle ist oder die Metalllegierung wenigstens ein Metall der nachstehenden Metalle enthält: Magnesium, Zink, Zirconium, Kohlenstoff, Eisen, Natrium, Kalzium, Mangan, Molybdän oder Selen. Auch ist es möglich, die Stentstreben 2 aus einem bioresorbierbaren Polymer der Familie der Poly- (L-Lactide), Poly- Lactid-co-Glycolide oder Poly-Glycolactide, Poly-Carbonate, Poly-Chitosane oder Phospholipide zu fertigen. Durch die Materialwahl sowie auch die Materialstärke, mit der die einzelnen Stentstreben 2 gefertigt sind, können die Auflösungseigenschaften im Wege der Bioresorption des Stents nach Implantation innerhalb des Körpers vorbestimmt werden. So ist es insbesondere möglich, die therapeutische Wirkdauer des implantierten Stents vorab zu definieren und festzulegen, d.h. die Anzahl in Jahren und/oder Monaten kann vorab festgelegt werden, ab wann der Stent 1 nach der Implantation seine therapeutische, das Hohlorgan aufweitende Wirkung verliert und sich letztlich im Wege der Bioresorption vollständig auflöst.

Im Unterschied zu bekannten, sich vollständig im Wege der Bioresorption auflösenden Stents weist der lösungsgemäß ausgebildete Stent einen auf der Mantelfläche M der röhrchenförmig oder hohlzylinderförmig ausgebildeten Raumform des Stents 1 befindlichen Flächenbereich 6 auf, der durch einen virtuellen oder reellen Umfangsrand 7 umgeben ist und den Flächenbereich 6 von den Stentstreben des übrigen Gittergerüstes des Stents 1 trennt. Im Falle des in Fig. 1a, b illustrierten Ausführungsbeispiels ist der Umfangsrand 7 oval ausgebildet. In Fig. 1b ist der Stent 1 in einem radial aufgeweiteten Zustand dargestellt, den der Stent 1 nach dem Vorgang der Implantation und radialen Dilatation einnimmt. Die innerhalb des Flächenbereiches 6 befindlichen Stentstreben 2' unterscheiden sich von den außerhalb des Flächenbereiches 6 befindlichen Stentstreben 2 dadurch, dass sie sich in einer kürzeren Zeitspanne nach intrakorporaler Implantation aufzulösen in der Lage sind, als die Stentstreben 2 außerhalb des Flächenanteils 6, und zwar derart, dass sich die Stentstreben 2' in zeitlicher Abfolge innerhalb des Flächenbereiches 6 von dessen Zentrum aus hin zum Umfangsrand 7 aufzulösen vermögen.

Die Fig. 1c stellt die Situation nach vollständiger Auflösung der innerhalb des Flächenbereiches 6 befindlichen Stentstreben 2' dar. Die durch den Umfangsrand 7 einbeschriebene Öffnung 8 dient dem freien Zugang zu einer Gefäßabzweigung von einem Hohlorgan, innerhalb dem der Stent 1 zu dessen radialen Aufweitung positioniert ist. Form und Größe der Öffnung 8 sind an die Öffnungsform und -weite der Gefäßabzweigung angepasst, so dass die im Bereich der Gefäßabzweigung vorherrschenden Strömungsverhältnisse nicht beeinträchtigt werden.
Längs des Umfangsrandes 7 sind röntgendichte Marker 11 angebracht, die einem Operateur eine exakte Positionierung des Stents 1 relativ zu einer Gefäßabzweigung ermöglichen.

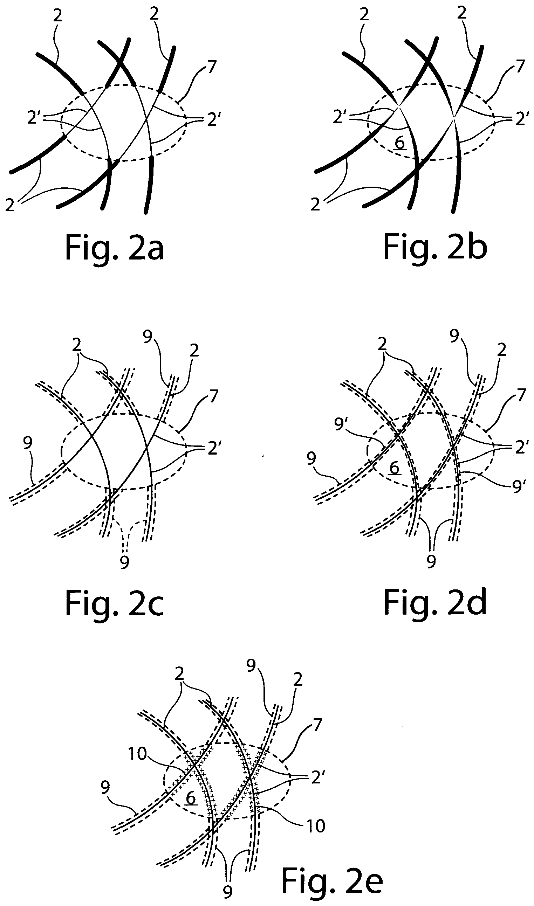

Je nach Anatomie der zu therapierenden Hohlorgane können in Abweichung zu dem in Fig. 1 illustrierten Ausführungsbeispiel mehrere Flächenbereiche 6 bzw. die sich daraus ergebenden freien Öffnungen 8 innerhalb eines Stents 1 vorgesehen werden. In den Fig. 2a bis e sind jeweils schematisierte Detaildarstellungen des durch den Umfangsrand 7 einbeschriebenen Flächenbereiches 6 dargestellt, anhand der alternative Maßnahmen erläutert werden, mit denen eine Auflösung der Stentstreben 2' innerhalb des Flächenbereiches 6 innerhalb einer kürzeren Zeitspanne realisierbar ist im Vergleich zur Zeitdauer, die nötig ist, um das Gittergerüst 3 des Stents 1 vollständig im Wege der Bioresorption aufzulösen.

Eine in Fig. 2a illustrierte erste Ausführungsform zeigt die Ausbildung von innerhalb des Flächenbereiches 6 befindlichen Stentstreben 2' mit einem geringeren Stentstrebendurchmesser im Vergleich zum Stentstrebendurchmesser der an den Umfangsrand 7 des Flächenbereiches 6 von außen angrenzenden Stentstreben 2. Aufgrund des geringeren Massenanteils pro Stentstrebenlänge der Stentstreben 2', die im übrigen aus dem gleichen bioresorbierbaren Material gefertigt sind wie die von außen an den Flächenanteil 6 angrenzenden Stentstreben 2, ist gewährleistet, dass die Stentstreben 2' nach kürzester Zeit, d.h. innerhalb weniger Wochen bzw. weniger Monate, vollständig aufgelöst sind und so zur freien Öffnung 8 führen. Der durch den Unterschied der Stentstrebendurchmesser bedingte Unterschied bezüglich des Massenanteils an bioresorbierbarem Material pro Strebenlänge kann zwischen den innerhalb des Flächenbereiches befindlichen Stentstreben 2' und den von außen an den Umfangsrand 7 des Flächenbereiches 6 angrenzenden Stentstreben 2 wenigstens 1 % bis maximal 60 % betragen.

Eine verbesserte, lösungsgemäße Ausführungsform gegenüber dem in Fig. 2a illustrierten Beispiel ist in Fig. 2b dargestellt. Auch in diesem Fall sind die Stentstrebendurchmesser der Stentstreben 2' gegenüber den Stentstreben 2 außerhalb des Flächenbereiches 6 kleiner ausgebildet, zusätzlich variiert ihr Stentstrebendurchmesser kontinuierlich derart, so dass der Stentstrebendurchmesser der Stentstreben 2' mit zunehmendem Abstand zum Umfangsrand 7 abnimmt. Der durch den Unterschied der Stentstrebendurchmesser bedingte Unterschied bezüglich des Massenanteils an bioresorbierbarem Material pro Strebenlänge kann zwischen radial entfernt zum Umfangsrand befindlichen Stentstreben 2' und Umfangsrand nahen Stenstreben 2' wenigstens 1 % bis maximal 60 % betragen. Durch diese Maßnahme ist gewährleistet, dass sich die innerhalb des Flächenbereiches 6 befindlichen Stentstreben 2' beginnend mit ihrem jeweils größten Abstand zum Umfangsrand 7 vollständig aufzulösen vermögen, bis schließlich die Stentstreben 2' durch Selbstauflösung im Wege der Bioresorption den Umfangsrand 7 erreicht haben und somit die freie Öffnung 8 bilden. Auf diese Weise kann ausgeschlossen werden, dass Teilablösungen von Stentstrebenabschnitten innerhalb des Flächenbereiches 6 erfolgen.

Eine weitere Möglichkeit für eine zeitlich kontrollierte Bioresorption des lösungsgemäß ausgebildeten Stents 1 ist in Fig. 2c illustriert. Hier sind die außerhalb des Flächenbereiches 6 befindlichen Stentstreben 2 mit einer ersten Materialschicht 9 beschichtet, wohingegen die innerhalb des Flächenbereiches 6 befindlichen Stentstreben 2' unbeschichtet und in der gleichen Weise wie in Figur 2b ausgebildet sind. Es liegt auf der Hand, dass die zusätzliche Materialschicht 9 zu einer Verlängerung der Bioresorption führt, so dass die unbeschichteten Stentstrebenabschnitte 2' innerhalb des Flächenbereiches 6 in einer kürzeren Zeitspanne vollständig resorbiert sind.

Eine weitere Ausführungsform sieht vor sowohl die Stentstreben 2 als auch die innerhalb des Flächenbereiches 6 befindlichen Stentstreben 2' mit einer Materialschicht aus einem einheitlichem Material zu beschichten, wobei die innerhalb des Flächenbereiches 6 befindlichen Stentstreben 2' mit einer dünneren Materialschichtdicke beschichtet sind als die Stentstreben 2 außerhalb des Flächenbereiches 6 (siehe Fig. 2d) und die dünnere Materialschicht 9' zudem eine kontinuierlich abnehmende Materialschichtdicke aufweist, je größer der Abstand längs der sich innerhalb des Flächenbereiches 6 befindlichen Stentstreben 2' zum Umfangsrand 7 wird. Durch die dünnere Schichtdickenausbildung der Materialschicht 9' auf den Stentstreben 2' innerhalb des Flächenbereiches 6, die 1 % bis maximal 60 % der Materialschichtdicke der Materialschicht 9 außerhalb des Flächenbereiches 6 betragen sollte, löst sich eben diese Materialschicht 9' in einer kürzeren Zeitspanne auf als im Falle der Materialschicht 9 auf den Stentstreben 2 außerhalb des Flächenbereiches 6, so dass wiederum gewährleistet ist, dass die Stentstreben 2' in einer kürzeren Zeitspanne resorbiert sind als die angrenzenden Stentstreben 2. Durch die zum Zentrum des Flächenbereiches 6 abnehmende Materialschichtdicke der Materialschicht 9' ist zudem gewährleistet, dass sich die Zentrumsnahen Stentstreben 2' innerhalb des Flächenbereiches 6 als Erstes aufzulösen beginnen, sodass die sich dabei ausbildende Öffnung innerhalb der Stentstreben 2'zum Umfangsrand 7 hin im Wege der andauernden Materialresorption radial ausweitet. Nicht notwendiger Weise, jedoch in vorteilhafter Form verjüngen sich die innerhalb des Flächenbereiches 6 befindlichen Stentstreben 2' mit zunehmenden Abstand zum Umfangsrand 7, wie im Ausführungsbeispiel gemäß Figur 2b erläutert. Denkbar ist in diesem Fall jedoch auch, dass sich durch die Gegenwart der zweiten Materialschicht 9' der beschriebene Effekt der sich radial von Innen nach Aussen ausweitenden Öffnung auch einstellt, sofern die Stentstreben 2' über einen einheitlichen dünnen Stentstrebendurchmesser verfügen.

Durch die vorzugsweise gleichmäßig erfolgende radiale Aufweitung der Stentstrebenöffnung ist es unmöglich, dass sich Einzelteile aus dem Stentstrebenverbund abtrennen und als Fremdkörper in die Blutbahn gelangen und dort unkontrolliert umhervagabundieren können. Gleichfalls ist es möglich, zusätzlich zur Dickenwahl der jeweils auf den Stentstreben 2, 2' aufzubringenden Materialschichten auch unterschiedliche Schichtmaterialien auf den Stentstreben 2, 2' aufzubringen, wobei darauf zu achten ist, dass sich das auf den Stentstreben 2' innerhalb des Flächenbereiches 6 aufgebrachte Schichtmaterial 10 schneller resorbierbar ist als das Schichtmaterial 9 auf den Stentstreben 2 (siehe Fig. 2e). Zudem ist es vorteilhaft die wenigstens zweite bioresorbierbare Materialschicht 10 als bioresorbierbare Polymerschicht auszubilden, die wenigstens ein Medikament aufnimmt und freisetzt. Das wenigstens eine Medikament kann vorzugsweise aus der Gruppe der anti-proliferativen Substanzklasse, der Limusgruppe wie Sirolimus, Everolimus, Zotarolimus, der Substanzklasse der Statine, der P2Y12 Antagonisten oder der Thrombinantagonisten gewählt sein.

Die vorstehenden alternativen Ausgestaltungsmöglichkeiten zum Zweck einer zeitlich gestaffelten Auflösung der Stentstreben 2 und 2' können in geeigneter Weise kombiniert werden.

### Bezugszeichenliste

- 1: Stent
- 2: Stentstreben
- 2': Stentstreben innerhalb des Flächenbereiches
- 3: Gittergerüst
- 4: Verbindungsstelle
- 5: Maschenöffnung
- 6: Flächenbereich
- 7: Umfangsrand
- 8: Öffnung
- 9: Erste Materialschicht
- 9': Erste Materialschicht mit kleiner Materialschichtdicke
- 10: Zweite Materialschicht
- 11: Röntgendichter Marker

## Patentansprüche

1. Stent mit einem röhrchenförmigen, aus miteinander verbundenen Stentstreben bestehenden Gittergerüst (3), das gesamtheitlich aus einem bioresorbierbaren Material gefertigt ist und aus einer komprimierten ersten Raumform in eine radial aufgeweitete, formstabile röhrchenförmige zweite Raumform überführbar ist,
**dadurch gekennzeichnet,**
**dass** sämtliche Stentstreben des röhrchenförmigen Gittergerüstes (3) aus einem einheitlichen, bioresorbierbaren Material bestehen,
**dass** das röhrchenförmige Gittergerüst (3) wenigstens einen, auf einer dem Gittergerüst (3) zuordenbaren, zylinderförmigen Mantelfläche (M) befindlichen, einstückig zusammenhängenden Flächenbereich (6) der Stentstreben umfasst, die von an den Flächenbereich (6) mittel- und/oder unmittelbar angrenzenden Stentstreben (2) des Gittergerüstes (3) umgeben sind, derart aufweist,
**dass** die Stenstreben (2') innerhalb des Flächenbereiches (6) einen geringeren Massenanteil an bioresorbierbaren Material pro einer vorgebbaren diskreten Strebenlänge, im Weiteren kurz pro Strebenlänge, aufweisen als die mittel- und/oder unmittelbar an den Flächenbereich (6) angrenzenden Stentstreben (2) des Gittergerüstes (3),
**dass** der Flächenbereich (6) von einem den Flächenbereich (6) einbeschreibenden virtuellen oder durch einen durch eine Stentstrebenstruktur definierten, in sich geschlossenen Umfangsrand (7) umgeben ist, und
**dass** der Massenanteil an bioresorbierbarem Material pro Strebenlänge der Stentstreben (2') innerhalb des Flächenbereiches (6) mit zunehmendem Abstand der Stentstreben (2') zum Umfangsrand (7) kontinuierlich oder graduell abnimmt und/oder
**dass** die mittel- und/oder unmittelbar an den Flächenbereich (6) angrenzenden Stentstreben (2) des Gittergerüstes (3) mit wenigstens einer ersten bioresorbierbaren Materialschicht (9) und die Stentstreben (2') innerhalb des Flächenbereiches (6) mit wenigstens einer zweiten bioresorbierbaren Materialschicht (10) beschichtet sind, die in einem kürzeren Zeitraum bioresorbierbar ist, als die erste bioresorbierbare Materialschicht (9) und dass eine der wenigstens zweiten Materialschicht (10) zuordenbare Schichtdicke mit zunehmendem Abstand der mit der zweiten Materialschicht (10) beschichteten Stentstreben (2') zum Umfangsrand (7) kontinuierlich oder graduell abnimmt.

2. Stent nach Anspruch 1,
**dadurch gekennzeichnet, dass** das röhrchenförmige Gittergerüst (3) über eine einheitliche Gitterstruktur verfügt.

3. Stent nach Anspruch 1 oder 2,
**dadurch gekennzeichnet, dass** die Stentstreben (2. 2') einstückig miteinander verbunden sind und jeweils offene Gitterstrukturmaschen umschließen, und dass sämtliche Gitterstrukturmaschen über eine einheitliche Form verfügen.

4. Stent nach einem der Ansprüche 1 bis 3,
**dadurch gekennzeichnet, dass** der Umfangsrand (7) kreisförmig, elliptisch, oval ist oder eine n-eckige Form aufweist, mit n ≥ 3 und n ist eine natürliche Zahl.

5. Stent nach einem der Ansprüche 1 bis 4,
**dadurch gekennzeichnet, dass** die mittel- und/oder unmittelbar an den Flächenbereich (6) angrenzenden Stentstreben (2) des Gittergerüstes (3) mit wenigstens der ersten bioresorbierbaren Materialschicht (9) beschichtet sind und die Stentstreben (2') innerhalb des Flächenbereiches (6) unbeschichtet sind.

6. Stent nach einem der Ansprüche 1 bis 5,
**dadurch gekennzeichnet, dass** die Stentstreben (2') innerhalb des Flächenbereiches (6) kleinere Stentdurchmesser aufweisen, als die mittel- und/oder unmittelbar angrenzenden Stentstreben (2) des Gittergerüstes (3).

7. Stent nach einem der Ansprüche 1 bis 6,
**dadurch gekennzeichnet, dass** wenigstens zwei innerhalb des Flächenbereiches (6) befindliche Stentstreben (2') jeweils über einen Massenanteil an bioresorbierbaren Material pro Strebenlänge verfügen, die sich wenigstens um 1 % bis maximal 60 % voneinander unterscheiden, und/oder
dass wenigstens zwei innerhalb des Flächenbereiches (6) befindliche Stentstreben (2') mit der zweiten Materialschicht (10) beschichtet sind, deren zuordenbaren Schichtdicken sich wenigstens um 1 % bis maximal 60 % voneinander unterscheiden

8. Stent nach einem der Ansprüche 1 bis 7,
**dadurch gekennzeichnet, dass** längs des virtuellen Umfangsrandes (7) wenigstens ein röntgendichter Marker (11) an dem Gittergerüst (3) angebracht ist.

9. Stent nach einem der Ansprüche 1 bis 8,
**dadurch gekennzeichnet, dass** das bioresorbierbare Material ein Metall oder eine Metalllegierung ist,
dass das Metall eines der nachstehenden Metalle ist oder die Metalllegierung wenigstens ein Element der nachstehenden Elemente enthält: Magnesium, Zink, Zirconium, Kohlenstoff, Eisen, Natrium, Calcium, Mangan, Molybdän oder Selen.

10. Stent nach einem der Ansprüche 1 bis 9,
**dadurch gekennzeichnet, dass** das bioresorbierbare Material aus einem der bioresorbierbaren Polymere der Familie der Poly- (L-Lactide), Poly- Lactid-co-Glycolide oder Poly-Glycolactide, Poly-Carbonate, Poly-Chitosane oder Phospholipide besteht.

11. Stent nach einem der Ansprüche 1 bis 10,
**dadurch gekennzeichnet, dass** die wenigstens zweite bioresorbierbare Materialschicht (10) eine bioresorbierbare Polymerschicht ist, die wenigstens ein Medikament aufnimmt und freisetzt.

12. Stent nach Anspruch 11,
**dadurch gekennzeichnet, dass** das wenigstens eine Medikament aus der Gruppe der anti-proliferativen Substanzklasse der Limusgruppe wie Sirolimus, Everolimus, Zotarolimus, der Substanzklasse der Statine, der P2Y12 Antagonisten oder der Thrombinantagonisten gewählt ist.

13. Stent nach einem der Ansprüche 1 bis 12,
**dadurch gekennzeichnet, dass** die wenigstens eine zweite bioresorbierbare Materialschicht (10) aus dem gleichen bioresorbierbaren Material besteht, wie die erste Materialschicht (9), jedoch über eine geringere Schichtdicke verfügt als die erste bioresorbierbare Materialschicht (9).

## Claims

1. A stent with a tubular framework structure consisting of interconnected stent struts fabricated as a whole from a bioresorbable material and being convertible from a compressed first geometric shape into a radially dilated, dimensionally stable, tubular second geometric shape,
**characterized in that**
all the stent struts of the tubular framework structure are made of a uniform bioresorbable material;
the tubular framework structure comprises at least one surface region, which is contiguous in one piece and is situated on a lateral cylindrical surface that can be attributed to the framework structure, the surface region comprising the stent struts that are surrounded by stent struts of the framework structure that are directly and/or indirectly adjacent to the surface region, such that
the stent struts within the surface region have a smaller amount by mass of bioresorbable material per one predefinable discrete strut length, hereinafter referred to simply as per strut length, than the stent struts of the framework structure adjacent directly or indirectly to the surface regions,
the surface region is surrounded by a self-contained circumferential edge inscribing the surface region, the circumferential edge being virtual or defined by a stent strut structure, and
the amount by mass of bioresorbable material per strut length of the stent struts within the surface region decreases continuously or gradually with an increase in the distance of the stent struts from the circumferential edge and/or
the stent struts of the framework structure directly and/or indirectly adjacent to the surface region are coated with at least one first bioresorbable material layer, and the stent struts inside the surface region are coated with at least one second bioresorbable material layer, which is bioresorbable in a shorter period of time than the first bioresorbable material layer, and that a layer thickness that can be attributed to the at least one second material layer decreases continuously or gradually with an increase in the distance of the stent struts coated with the second material layer from the circumferential edge.

2. The stent according to Claim 1,
**characterized in that** the tubular framework structure has a uniform framework structure.

3. The stent according to Claim 1 or 2,
**characterized in that** the stent struts are connected to one another in one piece and each enclose open framework structure meshes, and all the framework structure meshes have a uniform shape.

4. The stent according to any one of Claims 1 to 3,
**characterized in that** the circumferential edge is circular, elliptical or oval or has an n-angular shape, wherein n ≥ 3 and n is a natural number.

5. The stent according to any one of Claims 1 to 4,
**characterized in that** the stent struts of the framework structure directly and/or indirectly adjacent to the surface region are coated at least with the one first bioresorbable material layer, and the stent struts inside the surface region are uncoated.

6. The stent according to any one of Claims 1 to 5,
**characterized in that** the stent struts within the surface region have smaller stent diameters than the stent struts of the framework structure directly and/or indirectly adjacent.

7. The stent according to any one of Claims 1 to 6,
**characterized in that** at least two stent struts situated inside the surface region each have an amount by mass of bioresorbable material per strut length differing from one another by at least 1% to max. 60%,
and/or
that at least two stent struts situated inside the surface region are coated with the second material layer, whose assignable layer thicknesses differ from one another by at least 1 % up to max. 60%.

8. The stent according to any one of Claims 1 to 7,
**characterized in that** at least one radiopaque marker is applied to the framework structure along the virtual circumferential edge.

9. The stent according to any one of Claims 1 to 8,
**characterized in that** the bioresorbable material is a metal or a metal alloy,
that the metal is one of the following metals or the metal alloy comprises at least one element of the following elements: magnesium, zinc, zirconium, carbon, iron, sodium, calcium, manganese, molybdenum or selenium.

10. The stent according to any one of Claims 1 to 9,
**characterized in that** the bioresorbable material consists of one of the bioresorbable polymers of the family of poly-(L-lactides), polylactide-co-glycolides or poly-glycolactides, polycarbonates, poly-chitosans or phospholipids.

11. The stent according to any one of Claims 1 to 10,
**characterized in that** the at least one second bioresorbable material layer is a bioresorbable polymer layer that holds and releases at least one medication.

12. The stent according to Claim 11,
**characterized in that** the at least one medication is selected from the group of the antiproliferative substance class of the limus group such as sirolimus, everolimus, zotarolimus, the substance class of statins, P2Y12 antagonists or thrombin antagonists.

13. The stent according to any one of Claims 1 to 12,
**characterized in that** the at least one second bioresorbable material layer consists of the same bioresorbable material as the first material layer but has a smaller layer thickness than the first bioresorbable material layer.

## Revendications

1. Stent comportant une structure grillagée en forme de tubule composées de tiges de stent reliées entre elles (3), qui est fabriquée en totalité dans un matériau biorésorbable et peut passée d'une première forme spatiale comprimée à une seconde forme spatiale élargie radialement de forme stable et en forme de tubule, **caractérisé en ce que**
toutes les tiges de stent de la structure grillagée en forme de tubule (3) sont composées d'un matériau uniforme et biorésorbable,
que la structure grillagée en forme de tubule (3) comprend au moins une zone superficielle (6) se trouvant sur une surface d'enveloppe (M) de forme cylindrique, associable à la structure grillagée (3) et en cohésion monobloc avec celle-ci (6) des tiges de stent qui sont entourées de tiges de stent (2) indirectement et/ou directement adjacentes à la zone superficielle (6) de la structure grillagée (3) de telle sorte
que les tiges de stent (2') présentent dans la zone superficielle (6) un plus faible pourcentage en masse de matériau biorésorbable par longueur discrète prescriptible de tige, appelée ci-après en abrégé longueur de tige, que les tiges de stent (2) indirectement et/ou directement adjacentes à la zone superficielle (6) de la structure grillagée (3),
que la zone superficielle (6) est entourée d'une bordure périphérique virtuelle (7) circonscrivant la zone superficielle (6) ou définie par une structure de tige de stent et refermée sur elle-même, et
que le pourcentage en masse de matériau biorésorbable par longueur de tige des tiges de stent (2') diminue en continu ou graduellement dans la zone superficielle (6) au fur et à mesure de l'augmentation de la distance entre les tiges de stent (2') et la bordure circonférentielle (7) et/ou
que les tiges de stent (2) indirectement et/ou directement adjacentes à la zone superficielle (6) de la structure grillagée (3) sont revêtues d'au moins une première couche de matériau biorésorbable (9) et que les tiges de stent (2') sont revêtues dans la zone superficielle (6) d'au moins une seconde couche de matériau biorésorbable (10) qui est résorbable sur une durée plus courte que la première couche de matériau biorésorbable (9) et qu'une épaisseur de couche associable à l'au moins une seconde couche de matériau (10) diminue en continu ou graduellement au fur et à mesure de l'augmentation de la distance entre la tige de stent (2') revêtus de la seconde couche de matériau (10) et la bordure circonférentielle (7).

2. Stent selon la revendication 1,
**caractérisé en ce que** la structure grillagée en forme de tubule (3) possède une structure grillagée uniforme.

3. Stent selon la revendication 1 ou 2,
**caractérisé en ce que** les tiges de stent (2. 2') sont raccordées entre elles en monobloc et renferment respectivement des mailles ouvertes de structure grillagée et que toutes les mailles de structure grillagée possèdent une forme uniforme.

4. Stent selon une des revendications 1 à 3, **caractérisé en ce que** la bordure périphérique (7) est circulaire, elliptique, ovale ou présente une forme à n angles, sachant que n ≥ 3 et que n est un nombre naturel.

5. Stent selon une des revendications 1 à 4,
**caractérisé en ce que** les tiges de stent (2) indirectement et/ou directement adjacentes à la zone superficielle (6) de la structure grillagée (3) sont revêtues au moins de la première couche de matériau biorésorbable (9) et que les tiges de stent (2') se trouvant dans la zone superficielle (6) ne sont pas revêtues.

6. Stent selon une des revendications 1 à 5,
**caractérisé en ce que** les tiges de stent (2') se trouvant dans la zone superficielle (6) présentent des diamètres de stent inférieurs à ceux des tiges de stent (2) indirectement et/ou directement adjacentes à la zone superficielle (6) de la structure grillagée (3).

7. Stent selon une des revendications 1 à 6,
**caractérisé en ce qu'**au moins deux tiges de stent (2') se trouvant dans la zone superficielle (6) comporte respectivement des proportions en masse de matériau biorésorbable par longueur de tige qui diffèrent d'au moins 1 % à 60 % au maximum l'une de l'autre, et/ou
qu'au moins deux tiges de stent (2') se trouvant dans la zone superficielle (6) sont revêtues de la seconde couche de matériau (10) dont les épaisseurs de couche associables diffèrent d'au moins 1 % à 60 % au maximum l'une de l'autre.

8. Stent selon une des revendications 1 à 7,
**caractérisé en ce que**, le long de la bordure circonférentielle virtuelle (7), au moins un marqueur hermétique aux rayons X (11) est apposé sur la structure grillagée (3).

9. Stent selon une des revendications 1 à 8,
**caractérisé en ce que** le matériau biorésorbable est un métal ou un alliage métallique,
le métal contient un des métaux suivants ou l'alliage métallique contient au moins un élément parmi les éléments suivants : magnésium, zinc, zirconium, carbone, fer, sodium, calcium, manganèse, molybdène ou sélénium.

10. Stent selon une des revendications 1 à 9,
**caractérisé en ce que** le matériau biorésorbable est composé d'un des polymères biorésorbables de la famille des poly-(L-lactides), polylactide-co-glycolides ou polyglycolactides, polycarbonates, polychitosanes ou phospholipides.

11. Stent selon une des revendications 1 à 10,
**caractérisé en ce que** l'au moins une seconde couche de matériau biorésorbable (10) est une couche de polymère biorésorbable qui absorbe et libère au moins un médicament.

12. Stent selon la revendication 11,
**caractérisé en ce que** l'au moins un médicament est sélectionné dans le groupe de la classe de substances anti-prolifératives du groupe Limus comme Sirolimus, Everolimus, Zotarolimus, de la classe de substances des statines, des antagonistes de P2Y12 ou des antagonistes de thrombine.

13. Stent selon une des revendications 1 à 12,
**caractérisé en ce que** l'au moins une seconde couche de matériau biorésorbable (10) est composée du même matériau biorésorbable que la première couche de matériau (9) mais possède une épaisseur de couche inférieure à celle de la première couche de matériau biorésorbable (9).
